(19) Europäisches Patentamt
European Patent Office
Office européen des brevets

(11) **EP 1 918 756 B1**

## (12) EUROPÄISCHE PATENTSCHRIFT

(45) Veröffentlichungstag und Bekanntmachung des Hinweises auf die Patenterteilung:
**28.07.2010  Patentblatt 2010/30**

(21) Anmeldenummer: **07019793.4**

(22) Anmeldetag: **10.10.2007**

(51) Int Cl.:
*G02B 21/06* (2006.01)     *A61B 19/00* (2006.01)
*G02B 21/00* (2006.01)     *A61B 5/00* (2006.01)
*G01B 9/02* (2006.01)

(54) **Operationsmikroskop mit OCT-System und Operationsmikroskop-Beleuchtungsmodul mit OCT-System**

Operation microscope with OCT system and operation microscope illumination module with OCT system

Microscope d'opération doté d'un système OCT et module d'éclairage de microscope d'opération doté d'un système OCT

(84) Benannte Vertragsstaaten:
**DE ES FR GB IT**

(30) Priorität: **06.11.2006  DE 102006052513**
        **24.04.2007  DE 102007019677**

(43) Veröffentlichungstag der Anmeldung:
**07.05.2008  Patentblatt 2008/19**

(60) Teilanmeldung:
**10155318.8**

(73) Patentinhaber: **Carl Zeiss Surgical GmbH**
**73447 Oberkochen (DE)**

(72) Erfinder:
• **Reimer, Peter**
**73479 Ellwangen (DE)**
• **Abele, Alfons**
**73527 Schwäbisch Gmünd (DE)**
• **Hauger, Christoph**
**73431 Aalen (DE)**
• **Seesselberg, Markus**
**73431 Aalen (DE)**

(74) Vertreter: **Gauss, Nikolai**
**c/o Carl Zeiss AG**
**Patentabteilung**
**Carl-Zeiss-Strasse 22**
**73447 Oberkochen (DE)**

(56) Entgegenhaltungen:
**EP-A- 0 815 801     EP-A- 0 941 692**
**WO-A- 03/070090**

**Beschreibung**

Die Erfindung betrifft ein Operationsmikroskop mit einem Beobachtungsstrahlengang zur Untersuchung eines Objektbereichs, mit einer Beleuchtungseinrichtung, die eine Beleuchtungsoptik umfasst, welche eine von einer Lichtquelle ausgeleuchtete Leuchtfeldblende zu einem parallelen Beleuchtungsstrahlengang nach unendlich abbildet, wobei die Beleuchtungsoptik eine erste Linsengruppe und eine zweite Linsengruppe aufweist, mit einem Objektiv, das im parallelen Beleuchtungsstrahlengang angeordnet ist, um die Leuchtfeldblende auf den Objektbereich abzubilden, und mit einem OCT-System zur Untersuchung des Objektbereichs, wobei das OCT-System einen OCT-Abtaststrahlengang umfasst, der durch das Objektiv geführt ist.

Die Erfindung betrifft weiter ein Operationsmikroskop-Beleuchtungsmodul zum Anschluss an ein Operationsmikroskop mit einer Aufnahme für einen ersten Lichtleiter, um Beleuchtungslicht bereitzustellen, mit einer Leuchtfeldblende, die mit Licht aus dem Lichtleiter ausgeleuchtet werden kann, mit einer Beleuchtungsoptik, welche die Leuchtfeldblende zu einem parallelen Abbildungsstrahlengang nach unendlich abbildet, wobei die Beleuchtungsoptik eine erste Linsengruppe und eine zweite Linsengruppe aufweist und mit einem Beleuchtungsspiegel, der dazu dient, das aus der Leuchtfeldblende austretende Beleuchtungslicht mit parallelem Abbildungsstrahlengang durch das Mikroskop-Hauptobjektiv zum Objektbereich zu lenken.

Ein Operationsmikroskop der eingangs genannten Art ist aus der EP 0 815 801 bekannt. Dieses Operationsmikroskop enthält ein OCT-System, das einen OCT-Abtaststrahlengang aus kurzkohärenter Laserstrahlung erzeugt. Das OCT-System enthält eine Analyseeinheit zur Auswertung von Interferenzsignalen. Es umfasst eine Einrichtung zum Scannen des OCT-Abtaststrahlengangs mit zwei Scanspiegeln, die um zwei Bewegungsachsen verstellt werden können. Der OCT-Abtaststrahlengang in dem Operationsmikroskop ist über einen Teilerspiegel in den Beleuchtungsstrahlengang des Operationsmikroskops eingekoppelt. Er wird mit diesem durch das Mikroskop-Hauptobjektiv hindurch zum Objektbereich gelenkt.

Ein Operationsmikroskop-Beleuchtungsmodul der eingangs genannten Art enthält das Carl Zeiss Operationsmikroskopsystem OPMI® Visu 200. Dieses Beleuchtungsmodul ist zur Befestigung an dem Grundkörper eines Operationsmikroskops ausgebildet. Es umfasst als Beleuchtungsoptik zwei Linsengruppen, die eine mit Licht aus dem Lichtleiter ausgeleuchtete Leuchtfeldblende in einen parallelen Abbildungsstrahlengang überführen, der senkrecht zur optischen Achse des Mikroskop-Hauptobjektivs verläuft, wenn das Beleuchtungsmodul an das Operationsmikroskop angeschlossen ist. Das Beleuchtungsmodul enthält zwei Beleuchtungsspiegel, die das Beleuchtungslicht parallel zur optischen Achse des Mikroskop-Hauptobjektivs umlenken.

Ein OCT-System erlaubt mittels optischer Kohärenztomographie die nichtinvasive Darstellung und Messung von Strukturen innerhalb eines Gewebes. Als optisches bildgebendes Verfahren ermöglicht die optische Kohärenztomographie insbesondere Schnitt- oder Volumenbilder von biologischem Gewebe mit Mikrometerauflösung zu erzeugen. Ein entsprechendes OCT-System umfasst eine Quelle für zeitlich inkohärentes und räumlich kohärentes Licht mit einer Kohärenzlänge $I_c$, die einem Probenstrahlengang und einem Referenzstrahlengang zugeführt wird. Der Probenstrahlengang ist auf das zu untersuchende Gewebe gerichtet. Laserstrahlung, die aufgrund von Streuzentren im Gewebe in den Probenstrahlengang zurückgestrahlt wird, überlagert das OCT-System mit Laserstrahlung aus dem Referenzstrahlengang. Durch die Überlagerung entsteht ein Interferenzsignal. Aus diesem Interferenzsignal lässt sich die Position von Streuzentren für die Laserstrahlung im untersuchten Gewebe bestimmen.

Für OCT-Systeme ist das Bauprinzip des "Time-Domain OCT" und des "Fourier-Domain OCT" bekannt.

Der Aufbau eines "Time-Domain OCT" ist beispielsweise in der US 5,321,501 anhand von Fig. 1a auf Sp. 5, Z. 40 - Sp. 11, Z. 10 beschrieben. In einem solchen System wird die optische Weglänge des Referenzstrahlenganges über einen schnell beweglichen Referenzspiegel fortlaufend variiert. Das Licht aus Proben- und Referenzstrahlengang wird auf einem Photodetektor überlagert. Wenn die optischen Weglängen von Proben- und Referenzstrahlengang übereinstimmen, entsteht auf dem Photodetektor ein Interferenzsignal.

Ein "Fourier-Domain OCT" ist beispielsweise in der WO 2006/100544 A1 erläutert. Um die optische Weglänge eines Probenstrahlenganges zu vermessen, wird wiederum Licht aus dem Probenstrahlengang Licht aus einem Referenzstrahlengang überlagert. Im Unterschied zu einem "Time-Domain OCT" wird jedoch für eine Messung der optischen Weglänge des Probenstrahlenganges das Licht aus Proben- und Referenzstrahlengang nicht direkt einem Detektor zugeführt, sondern zunächst mittels eines Spektrometers spektral zerlegt. Die so erzeugte spektrale Intensität des überlagerten Signals aus Proben- und Referenzstrahlengang wird dann mit einem Detektor erfasst. Durch Auswerten des Detektorsignals kann wiederum die optische Weglänge des Probenstrahlenganges ermittelt werden.

Aufgabe der Erfindung ist es, ein Operationsmikroskop mit OCT-System zu schaffen, das ein geringes Bauvolumen hat und dessen Bauprinzip ein einfaches Nachrüsten von Operationsmikroskopen für OCT ermöglicht, sowie ein Operationsmikroskop-Beleuchtungsmodul mit integriertem OCT-System zum Anschluss an ein Operationsmikroskop bereitzustellen.

Diese Aufgabe wird durch ein Operationsmikroskop der eingangs genannten Art gelöst, bei dem im Beleuchtungsstrahlengang zwischen der ersten Linsengruppe und der zweiten Linsengruppe ein Einkoppelelement vorgesehen ist, das den OCT-Abtaststrahlengang in den Beleuchtungsstrahlengang einkoppelt, sowie ein Operationsmikroskop-Beleuchtungsmodul der eingangs genannten Art gelöst, bei dem eine Aufnahme für einen zweiten Lichtleiter eines OCT-Systems vorgesehen ist, um einen OCT-Abtaststrahlengang bereitzustellen, wobei das Operationsmikroskop-Beleuchtungsmodul ein zwischen der ersten Linsengruppe und der zweiten Linsengruppe angeordnetes Einkoppelelement umfasst, das den OCT-Abtaststrahlengang in den Beleuchtungsstrahlengang einkoppelt.

In Weiterbildung der Erfindung ist bei dem Operationsmikroskop bzw. dem Operationsmikroskop-Beleuchtungsmodul das Einkoppelelement als Teilerspiegel, insbesondere als Planspiegel oder Teilerwürfel ausgebildet.

In Weiterbildung der Erfindung ist bei dem Operationsmikroskop das im parallelen Beleuchtungsstrahlengang angeordnete Objektiv als Mikroskop-Hauptobjektiv ausgebildet und wird von dem Beobachtungsstrahlengang des Operationsmikroskops durchsetzt. Auf diese Weise wird eine besonders kompakte Bauform des Operationsmikroskops erzielt.

In Weiterbildung des Operationsmikroskops ist auf der dem Objekt abgewandten Seite des Objektivs eine Umlenkeinrichtung für Beleuchtungslicht vorgesehen, die das Beleuchtungslicht der Beleuchtungseinrichtung zum Objektiv hin umlenkt. Auf diese Weise ist eine zu den Beobachtungsstrahlengängen achsnahe Beleuchtung möglich, die für ophthalmologische Operationen von Vorteil sein kann.

In Weiterbildung der Erfindung ist die Umlenkeinrichtung als Strahlenteiler ausgebildet, der von dem Beobachtungsstrahlengang des Operationsmikroskops durchsetzt ist. Auf diese Weise kann Beleuchtungslicht in den Beobachtungsstrahlengängen des Operationsmikroskops zum Objektbereich geführt werden.

In Weiterbildung der Erfindung enthält das Operationsmikroskop ein OCT-System für das Scannen des OCT-Abtaststrahlengangs mit einem ersten Scanspiegel. Vorzugsweise ist zusätzlich ein zweiter Scanspiegel vorgesehen, wobei der erste Scanspiegel um eine erste Drehachse bewegt werden kann und sich der zweite Scanspiegel um eine zweite Drehachse bewegen lässt, und wobei die erste Drehachse und die zweite Drehachse seitlich versetzt in einem rechten Winkel zueinander stehen. Auf diese Weise ist ein Abscannen des Objektbereichs mit senkrecht verlaufendem Rastermuster möglich.

In Weiterbildung der Erfindung umfasst das Operationsmikroskop ein OCT-System mit einem Lichtleiter der einen Lichtaustrittsabschnitt für den OCT-Abtaststrahlengang hat, der bewegbar gehalten ist. Auf diese Weise kann eine OCT-Abtastebene im Objektbereich variiert werden und es ist möglich, das System für unterschiedliche OCT-Wellenlängen in anbetracht der für sichtbares Licht ausgelegten optischen Komponenten im Beobachtungsstrahlengang einzustellen.

In Weiterbildung der Erfindung umfasst das Operationsmikroskop ein OCT-System mit verstellbarer Kollimationsoptik, welche den OCT-Abtaststrahlengang mit der zweiten Linsengruppe der Beleuchtungsoptik in einen im wesentlichen parallelen Abtaststrahlengang überführt. Auf diese Weise ist es möglich, die OCT-Abtastebene bei dem Operationsmikroskop relativ zur Beobachtungsebene der optischen Beobachtungsstrahlengänge des Systems zu verlagern.

Vorzugsweise umfasst das Operationsmikroskop-Beleuchtungsmodul eine Einrichtung zum Scannen des OCT-Abtaststrahlengangs. Diese Einrichtung zum Scannen kann beispielsweise einen ersten Scanspiegel und einen zweiten Scanspiegel aufweisen. Indem der erste Scanspiegel um eine erste Drehachse bewegt werden kann und der zweite Scanspiegel sich um eine zweite Drehachse verstellen lässt, wobei die erste Drehachse und die zweite Drehachse seitlich versetzt in einem rechten Winkel zueinander stehen, kann ein Objektbereich mit senkrecht verlaufendem Rastermuster abgescannt werden.

In Weiterbildung der Erfindung ist der Lichtaustrittsabschnitt des Lichtleiters für den OCT-Abtaststrahlengang im Operationsmikroskop-Beleuchtungsmodul bewegbar gehalten, um so die OCT-Abtastebene einstellen zu können.

Indem im Operationsmikroskop-Beleuchtungsmodul eine verstellbare Kollimationsoptik vorgesehen ist, mittels derer der OCT-Abtaststrahlengang mit der zweiten Linsengruppe der Beleuchtungsoptik in einen im wesentlichen parallelen Abtaststrahlengang überführt werden kann, ist es möglich, die OCT-Abtastebene des OCT-Systems relativ zu einer Beobachtungsebene für optische Beobachtungsstrahlengänge zu verlagern.

Vorteilhafte Ausführungsformen der Erfindung sind in den Figuren dargestellt und werden nachfolgend beschrieben.

Es zeigen:

Fig. 1 ein Operationsmikroskop mit Beleuchtungsmodul, in das ein OCT-System integriert ist;

Fig. 2 einen Schnitt des Mikroskop-Hauptobjektivs entlang der Linie II - II aus Fig. 1;

Fig. 3 einen Abschnitt des Beleuchtungsmoduls mit OCT-System;

Fig. 4 eine Intensitätsverteilung des aus dem Lichtleiter des OCT-Systems im Operationsmikroskop austretenden OCT-Abtastlichtstrahls; und

Fig. 5 eine Intensitätsverteilung des OCT-Abtastlichtstrahls in der OCT-Abtastebene im Objektbereich des Operationsmikroskops.

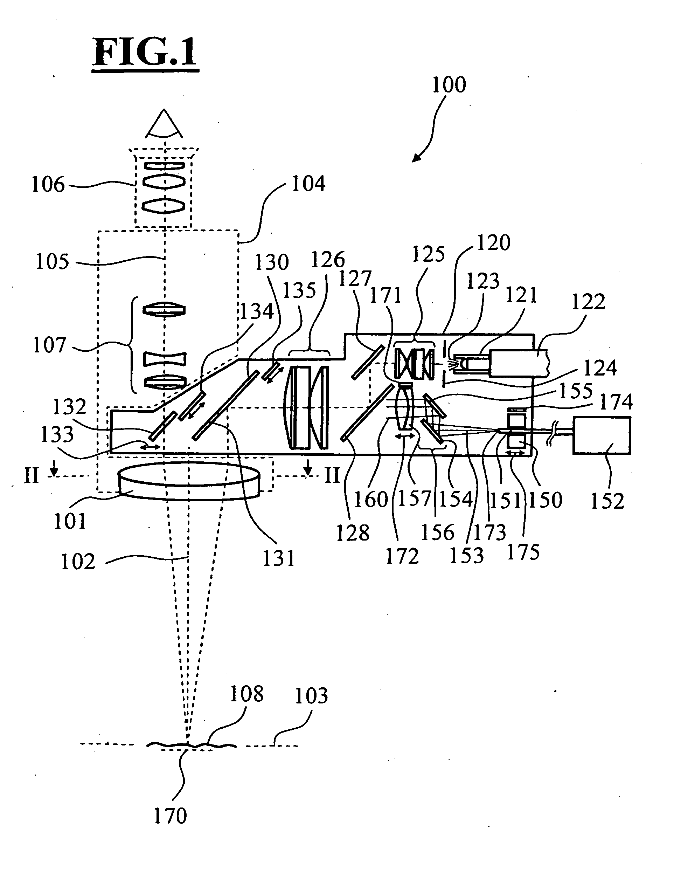

Das Operationsmikroskop 100 in Fig. 1 hat ein Mikroskop-Hauptobjektiv 101 mit einer optischen Achse 102 sowie eines Fokusebene 103, das in einem Operationsmikroskop-Grundkörper 104 aufgenommen ist. Das Mikroskop-Hauptobjektiv 101 wird von stereoskopischen Beobachtungsstrahlengängen 105 eines Binokulartubus 106 durchsetzt. Das Operationsmikroskop 100 enthält ein zoombares Vergrößerungssystem 107.

Zur Beleuchtung des Objektbereichs 108 hat das Operationsmikroskop 100 als Beleuchtungseinrichtung ein Beleuchtungsmodul 120. Dieses Beleuchtungsmodul 120 enthält eine Aufnahme 121 für einen ersten Lichtleiter 122, der Beleuchtungslicht 123 von einer nicht weiter dargestellten Lichtquelle bereitstellt. Mit dem aus dem ersten Lichtleiter 122 austretenden Beleuchtungslicht 123 wird eine verstellbare Leuchtfeldblende 124 ausgeleuchtet. In dem Beleuchtungsmodul 120 ist eine Beleuchtungsoptik angeordnet. Die Beleuchtungsoptik umfasst eine erste Linsengruppe 125, eine zweite Linsengruppe 126, sowie vier Spiegelelemente 127, 128, 129 und 130.

Das Spiegelelement 127 lenkt das aus der ersten Linsengruppe 125 austretende Beleuchtungslicht zu dem Spiegelelement 128, von wo es zu der zweiten Linsengruppe 126 gelangt. Die erste Linsengruppe 125 und die zweiten Linsengruppe 126 bilden die Leuchtfeldblende 124 nach unendlich ab. Somit tritt aus der zweiten Linsengruppe 126 Beleuchtungslicht 123 mit parallelem Strahlengang. Die optische Achse 120 des Beleuchtungsstrahlengangs verläuft austrittsseitig der zweiten Linsengruppe 126 senkrecht zur optischen Achse 102 des Mikroskop-Hauptobjektivs 101. Es wird auf einen Beleuchtungsspiegel 130 geführt, der eine Durchbrechung 131 aufweist. Der Beleuchtungsspiegel 130 lenkt das Beleuchtungslicht parallel zur optischen Achse 102 des Mikroskop-Hauptobjektivs 101 zum Objektbereich 108.

Das Beleuchtungsmodul 120 umfasst weiter einen Beleuchtungsspiegel 132 dem Beleuchtungslicht zugeführt wird, das durch die Durchbrechung 131 in den Beleuchtungsspiegel 130 gelangt.

Der Beleuchtungsspiegel 132 kann entsprechend dem Doppelpfeil 133 senkrecht zur optischen Achse 102 des Mikroskop-Hauptobjektivs 101 verstellt werden. Dies ermöglicht, den Einfallswinkel für Beleuchtungslicht im Objektbereich 108 einzustellen.

Zur Variation der Intensität des im Objektbereich geführten Beleuchtungslichts sind den Beleuchtungsspiegeln 130, 132 verstellbare Blenden 134, 135 zugeordnet. Durch Verstellen der Blenden 134, 135 kann die Intensität des durch das Mikroskop-Hauptobjektiv 101 geführten Beleuchtungslichts variiert werden.

Das Beleuchtungsmodul 120 weist eine Aufnahme 150 für einen zweiten Lichtleiter 151 auf, der mit einem OCT-System 152 verbunden ist.

Das OCT-System 152 ermöglicht zur Untersuchung des Objektbereichs 108 die Aufnahme von OCT-Bildern. Es umfasst eine Einheit für die Erzeugung und Analyse eines OCT-Abtaststrahlengangs 153.

Das OCT-System 152 ist vorzugsweise in eine nicht weiter dargestellte Stativkonsole des Operationsmikroskops integriert. Grundsätzlich könnte es aber auch in dem Beleuchtungsmodul 120 aufgenommen werden.

Der aus dem Lichtleiter 151 austretende Abtaststrahlengang 153 wird auf einen ersten Scanspiegel 154 und einen zweiten Scanspiegel 155 einer OCT-Scaneinheit 156 geführt. Er durchtritt nach der OCT-Scaneinheit 156 eine Sammellinse 157.

Das Strahlenbündel 160 aus der OCT-Scaneinheit 156 wird zu dem Spiegelelement 128 geführt. Das Spiegelelement 128 wirkt als Teilerspiegel. Es reflektiert das aus dem Lichtleiter 122 austretende Beleuchtungslicht nahezu vollständig, ist dabei jedoch für den OCT-Abtaststrahlengang durchlässig. Damit wird der OCT-Abtaststrahlengang 153 dem Beleuchtungslicht 123 überlagert. Das Spiegelelement 128 ist im Beleuchtungsmodul 120 als Spiegelelement mit Planplatten ausgeführt. Es könnte aber auch als Teilerwürfel ausgebildet werden.

Das Licht des OCT-Abtaststrahlengangs 153 wird über die Beleuchtungsspiegel 130, 132 mit dem Beleuchtungslicht zum Objektbereich 108 geführt. Die Sammellinse 157, die zweite Linsengruppe 126 der Beleuchtungsoptik im Beleuchtungsmodul 120 und das Mikroskop-Hauptobjektiv 101 bündeln den OCT-Abtaststrahlengang 153 in einer OCT-Abtastebene 170. Die OCT-Abtastebene 170 ist die Ebene der durch die optischen Elemente im OCT-Abtaststrahlengang mit OCT-Scaneinheit 156, Sammellinse 157, Spiegelelement 128, Spiegelelement 130 sowie Spiegelelement 132 und Mikroskop-Hauptobjektiv 101 festgelegten geometrischen Abbildung des Austrittsendes von Lichtleiter 173 in den Objektbereich 108. D.h., das entsprechende geometrische Bild des Lichtleiter-Austrittsendes liegt in der OCT-Abtastebene 170. Zur Einstellung der OCT-Abtastebene ist einerseits die Sammellinse 152 mittels eines Verstellantriebs 171 entsprechend dem Doppelpfeil 172 bewegbar gehalten. Andererseits kann hierzu das Austrittsende 173 des Lichtleiters 151 für den OCT-Abtaststrahlengang mittels einer Verstellvorrichtung 174 entsprechend dem Doppelpfeil 175 verlagert werden. Bei einem Verstellen der OCT-Abtastebene 170 wird der Referenzstrahlengang im OCT-System soweit erforderlich nachjustiert.

Das in den OCT-Abtaststrahlengang zurückgestreute Licht gelangt über das Mikroskop-Hauptobjektiv 101, die Spiegelelemente 132, 130, die Linsengruppe 126 und das Spiegelelement 128 zurück in das OCT-System 152. Dort wird das aus dem Objektbereich zurückgestreute OCT-Abtastlicht mit OCT-Strahlung aus einem Referenzstrahlengang interferiert. Das Interferenzsignal wird mittels eines Detektors erfasst und durch eine Rechnereinheit ausgewertet, die aus diesem Signal eine optische Weglängendifferenz zwischen Streuzentren für OCT-Licht im Objektbereich und der Weglänge von Licht im Referenzzweig bestimmt.

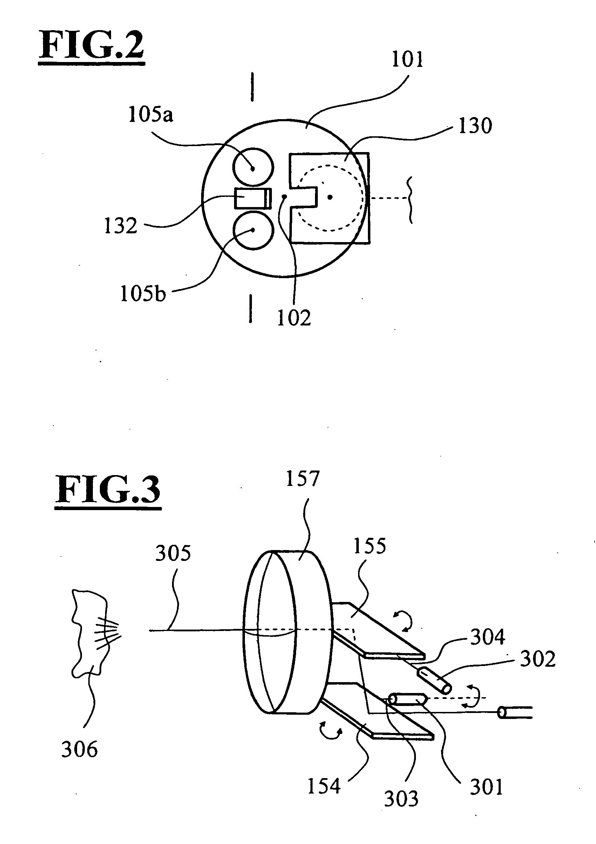

Fig. 2 ist ein Schnitt entlang der Linie II - II aus Fig. 1. Diese Figur zeigt die Beleuchtungsspiegel 130, 132 und erläutert den Verlauf der stereoskopischen Beobachtungsstrahlengänge 105 im Operationsmikroskop 100 aus Fig. 1. Das Mikroskop-Hauptobjektiv 101 wird von zwei stereoskopischen Teilstrahlengängen 105a, 105b durchsetzt. Die optische Achse 102 des Mikroskop-Hauptobjektivs 101 liegt in dessen Zentrum.

Fig. 3 zeigt die OCT-Scaneinheit 156 des Operationsmikroskops 100 aus Fig. 1. Der erste Scanspiegel 154 und der zweite Scanspiegel 155 sind mittels Stellantrieben 301, 302 um zwei zueinander senkrecht verlaufende Achsen 303, 304 drehbeweglich angeordnet. Dies ermöglicht, den OCT-Abtaststrahlengang 305 über eine Ebene 306 zu scannen.

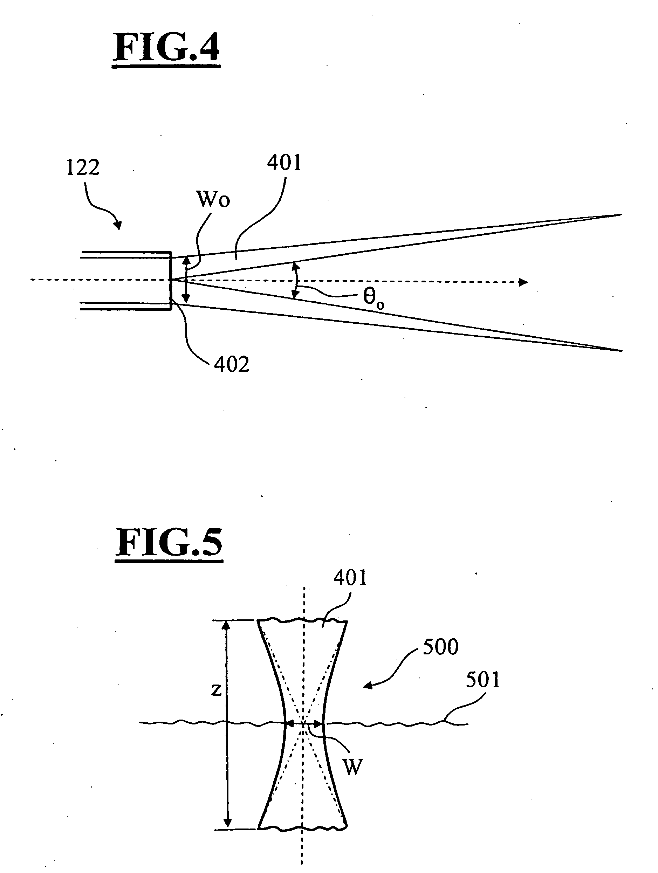

Fig. 4 zeigt den Frontabschnitt 402 des Lichtleiters 151 aus Fig. 1. Der Lichtleiter 151 wirkt für Licht der Wellenlänge λ = 1310nm als Monomodenfaser. Der Durchmesser d des Faserkerns des Lichtleiters 122 genügt der Beziehung

$$\frac{d}{2} < 2.4 \frac{\lambda}{2\pi NA},$$

wobei NA die numerische Apertur der Frontfläche des Lichtleiters ist. Vorzugsweise liegt der Durchmesser d des Faserkerns des Lichtleiters 122 im Bereich 5 $\mu$m < d < 10$\mu$m. In diesem Parameterbereich führt der Lichtleiter 122 das Licht mit gaussförmigen Wellenmoden. Aus dem Lichtleiter 151 tritt der OCT-Abtastlichtstrahl 401 mit einem näherungsweise gaussförmigen Strahlungsprofil aus, das durch einen Taillenparamter Wo und einem Öffnungsparameter θ$_0$ charakterisiert ist, wobei gilt:

$$\theta_0 = \frac{2\lambda}{\pi W_0}$$

Für einen Faserkerndurchmesser von d$_0$ = 10$\mu$m, einen Wellenlänge λ$_0$ = 1310 nm ergibt sich damit als Maß für die Strahldivergenz ein Öffnungswinkel von θ$_0$ ≈ 0,0827 rad.

Die Frontfläche 402 des Lichtleiters 151 wird über die Scanspiegel 154 und 155 im Operationsmikroskop 100 aus Fig. 1, die Sammellinse 157, das Spiegelelement 128, die zweite Linsengruppe 126, das Spiegelelement 130 und das Spiegelelement 132 durch das Mikroskop-Hauptobjektiv 101 auf den Objektbereich 107 in die OCT-Abtastebene 170 abgebildet.

Die Fig. 5 zeigt den Verlauf der Intensitätsverteilung des OCT-Abtastlichtstrahls 401 senkrecht zur OCT-Abtastebene 501. In der OCT-Abtastebene 501 hat die Intensitätsverteilung der OCT-Abtaststrahlung eine kleinste Einschnürung. Außerhalb der OCT-Abtastebene nimmt der Durchmesser des OCT-Abtaststrahlengangs zu. Da der OCT-Abtastlichtstrahl 401 aus dem Lichtleiter 151 aus Fig. 4 mit einem näherungsweise gaussförmigen Strahlungsprofil austritt, bewirken die Sammellinse 157 und das Mikroskop-Hauptobjektiv 101 für den OCT-Abtastlichtstrahl im Bereich der OCT-Abtastebene 170 ein sogenanntes Gaussbündel 500 des OCT-Abtastlichtstrahls 401. Dieses Gaussbündel 500 ist durch den konfokalen Parameter z als Maß für die longitudinale Ausdehnung der Taille des Gaussbündels, sowie den Taillenparameter W als Maß für den Durchmesser der kleinsten Einschnürung 502 des OCT-Abtastlichtstrahls 401, d.h. für den Durchmesser von dessen Taille charakterisiert, wobei gilt:

$$z = 2 \frac{W^2 \pi}{\lambda},$$

dabei ist λ die Wellenlänge des OCT-Abtastlichtstrahls. Zwischen dem Taillenparametern W des Gaussbündels 500 und dem Taillenparameter Wo des in Fig. 4 gezeigten, aus dem Lichtleiter 151 austretenden Abtastlichtstrahls 401 gilt die folgende Beziehung:

$$W = \beta W_0,$$

wobei β der Vergrößerungs- bzw. Verkleinerungsparameter der oben erwähnten geometrischen Abbildung des Austrittsendes von Lichtleiter 151 aus Fig. 1 in der OCT-Abtastebene ist. β ist mit der Brennweite f$_1$ der Sammellinse 157 aus Fig. 1 und der Brennweite f$_2$ des Mikroskop-Hauptobjektivs über die folgende Beziehung verknüpft:

$$\frac{f_2}{f_1} = \beta$$

Die Größe von Strukturen, die sich mit dem OCT-Abtastlichtstrahl 401 auflösen lassen, ist durch dessen Durchmesser in der OCT-Abtastebene 170, d.h. durch den Taillenparameter W bestimmt. Erfordert beispielsweise eine Applikation eine Lateralauflösung des OCT-Systems im Operationsmikroskop von ca. 40$\mu$m, muss nach dem Nyquist-Theoren der Querschnitt des OCT-Abtastlichtstrahls 401 auf der Oberfläche ca. 20$\mu$m betragen. Bei gegebener Wellenlänge λ für den OCT-Abtastlichtstrahl 153 aus Fig. 1 müssen daher für eine gewünschte Auflösung des OCT-Systems 152 die Vergrößerung der optischen Abbildung im OCT-Strahlengang und der Durchmesser des Faserkerns im Lichtleiter 151 geeignet gewählt werden.

Der konfokale Parameter z als Maß für die longitudinale Ausdehnung der Taille des Gaussbündels bestimmt den axialen Tiefenbereich, aus dem in dem OCT-Abtaststrahlengang 153 aus Fig. 1 zurückgestreutes Licht detektiert werden kann: Je kleiner der konfokale Parameter z, desto größer ist der Verlust des OCT-Systems an lateraler Auflösung bei Entfernung eines mit OCT-Abtaststrahlung abgetasteten Objekts von der OCT-Abtastebene 170, da sich der Ort von Streuzentren nur innerhalb des durch den Taillenparamter W und den konfokalen Parameter z definierten "Trichter" lokalisieren lässt.

Nachdem die axiale Auflösung eines OCT-Systems einerseits durch die Kohärenzlänge $l_c$ des Lichts der im OCT-System eingesetzten Lichtquelle begrenzt ist, andererseits die laterale Auflösung des OCT-Systems abnimmt, wenn dessen Tiefenhub die mit dem konfokalen Parameter z gegebene Ausdehnung übersteigt, ist das Einstellen des konfokalen Parameters z auf den Tiefenhub des OCT-Systems günstig.

Für eine bestimmte Wellenlänge λ des OCT-Abtastlichtstrahls 401 ergibt sich dann die mögliche laterale Auflösung des OCT-Systems aus Fig. 1, da die Wellenlänge λ und der konfokale Parameter z den Taillenparameter W festlegen. Die Optikeinheiten im OCT-Abtaststrahlengang 153 aus Fig. 1 und die Bemassung des Faserkerns von Lichtleiter 151 sind dann so zu wählen, dass sich der betreffende Taillenparameter W ergibt.

Das Operationsmikroskop 100 ist so ausgelegt, dass die Fokusebene 170 des Mikroskop-Hauptobjektivs 101 für den sichtbaren Spektralbereich und OCT-Abtastebene 160 zusammenfällt. Dann liegt die in Fig. 5 gezeigte Taille 502 des OCT-Abtastlichstrahls in der Fokusebene des Operationsmikroskops.

Alternativ zu dieser Auslegung des Operationsmikroskops kann auch ein Versatz von OCT-Abtastebene und Fokusebene des Operationsmikroskops vorgesehen sein. Vorzugsweise ist dieser Versatz nicht größer als der konfokale Parameter z des OCT-Abtastlichtstrahls im Bereich der OCT-Abtastebene. Dies ermöglicht es beispielsweise einen unmittelbar unter der Fokusebene des Operationsmikroskops liegenden Objektbereich mittels OCT zu visualisieren. Es kann aber auch sinnvoll sein, für bestimmte Anwendungen einen definierten Versatz vorzusehen, der den konfokalen Parameter übersteigt, etwa um mit dem Operationsmikroskop die Vorderseite der Cornea eines Patientenauges untersuchen zu können und gleichzeitig mittels des OCT-Systems die Rückseite der Cornea des Patientenauges oder dessen Linse zu visualisieren.

Indem die OCT-Abtastebene um den Rayleighparameter z weiter vom Mikroskop-Hauptobjektiv 101 aus Fig. 1 entfernt ist, lässt sich der Tiefenhub für das OCT-System im Objektbereich maximieren.

Eine modifizierte Ausführungsform des anhand von Fig. 1 erläuterten Operationsmikroskops 100 enthält ein fokussierbares Mikroskop-Hauptobjektiv mit einstellbarer Brennweite. Auch diese Maßnahme ermöglicht das Verlagern einer OCT-Abtastebene und das Verändern der geometrischen Abbildung des Lichtleiter-Austrittsendes in die OCT-Abtastebene.

**Patentansprüche**

1. Operationsmikroskop (100)

   - mit einem Beobachtungsstrahlengang (105) zur Untersuchung eines Objektbereichs (108);
   - mit einer Beleuchtungseinrichtung (120), die eine Beleuchtungsoptik umfasst, welche eine von einer Lichtquelle ausgeleuchtete Leuchtfeldblende (124) zu einem parallelen Beleuchtungsstrahlengang nach unendlich abbildet;
   - wobei die Beleuchtungsoptik eine erste Linsengruppe (125) und eine zweite Linsengruppe (126) aufweist;
   - mit einem Objektiv (101), das im parallelen Beleuchtungsstrahlengang angeordnet ist, um die Leuchtfeldblende (124) auf den Objektbereich (108) abzubilden;
   - mit einem OCT-System (152) zur Untersuchung des Objektbereichs (108);
   - wobei das OCT-System (152) einen OCT-Abtaststrahlengang (153) umfasst, der durch das Objektiv (101) gerührt ist;
   **dadurch gekennzeichnet, dass**
   - im Beleuchtungsstrahlengang zwischen der ersten Linsengruppe (125) und der zweiten Linsengruppe (126) ein Einkoppelement (128) vorgesehen ist, das den OCT-Abtaststrahlengang in den Beleuchtungsstrahlengang einkoppelt.

2. Operationsmikroskop nach Anspruch 1, **dadurch gekennzeichnet, dass** das Einkoppelelement (128) als Teilerspiegel, insbesondere als Planspiegel oder Teilerwürfel ausgebildet ist.

3. Operationsmikroskop nach Anspruch 1 oder 2, **dadurch gekennzeichnet, dass** das im parallelen Beobachtungsstrahlengang angeordnete Objektiv als Mikroskop-Hauptobjektiv (101) ausgebildet ist und von dem Beobachtungsstrahlengang (105, 105a, 105b) des Operationsmikroskops (100) durchsetzt wird.

4. Operationsmikroskop nach Anspruch 3, **dadurch gekennzeichnet, dass** auf der objektabgewandten Seite des Objektivs (101) eine Umlenkeinrichtung (129, 130) vorgesehen ist, die das Beleuchtungslicht der Beleuchtungseinrichtung zum Objektiv (101) umlenkt.

5. Operationsmikroskop nach Anspruch 4, **dadurch gekennzeichnet, dass** die Umlenkeinrichtung als Strahlenteiler ausgebildet ist, der von dem Beobachtungsstrahlengang des Operationsmikroskops durchsetzt ist.

6. Operationsmikroskop nach einem der Ansprüche 1 bis 5, **dadurch gekennzeichnet, dass** das OCT-System für das Scannen des OCT-Abtaststrahlengangs einen ersten Scanspiegel (154) umfasst, der um eine erste Drehachse (303) bewegt werden kann.

7. Operationsmikroskop nach Anspruch 6, **dadurch gekennzeichnet, dass** ein zweiter Scanspiegel (155) vorgesehen ist, der um eine zweite Drehachse (304) bewegt werden kann, wobei die erste Drehachse (303) und die zweite Drehachse (304) seitlich versetzt in einem rechten Winkel zueinander stehen.

8. Operationsmikroskop nach einem der Ansprüche 1 bis 7, **dadurch gekennzeichnet, dass** das OCT-System einen Lichtleiter (151) umfasst, der einen Lichtaustrittsabschnitt für den OCT-Abtastrahlengang aufweist, wobei Mittel zum Bewegen des Lichtaustrittsabschnitts (174) des Lichtleiters (151) vorgesehen sind.

9. Operationsmikroskop nach einem der Ansprüche 1 bis 8, **dadurch gekennzeichnet, dass** das OCT-System eine Kollimationsoptik (157) umfasst, welche den OCT-Abtaststrahlengang mit der zweiten Linsengruppe (126) der Beleuchtungsoptik in einen im wesentlichen parallelen Abtaststrahlengang überführt.

10. Operationsmikroskop nach Anspruch 9, **dadurch gekennzeichnet, dass** für das Einstellen der OCT-Abtastebene (170) des OCT-Systems (152) Mittel zum Bewegen (171) der Kollimationsoptik (157) vorgesehen sind.

11. Operationsmikroskop-Beleuchtungsmodul (120) zum Anschluss an ein Operationsmikroskop (100)

    - mit einer Aufnahme (121) für einen ersten Lichtleiter (122) um Beleuchtungslicht (123) bereitzustellen;
    - mit einer Leuchtfeldblende (124), die mit Licht aus dem Lichtleiter (122) ausgeleuchtet werden kann;
    - mit einer Beleuchtungsoptik, welche die Leuchtfeldblende (120) zu einem parallelen Abbildungsstrahlengang nach unendlich abbildet; wobei
    - die Beleuchtungsoptik eine erste Linsengruppe (125) und eine zweite Linsengruppe (126) aufweist; und
    - mit einem Beleuchtungsspiegel (129, 130), der dazu dient, dass aus der Leuchtfeldblende (124) austretende Beleuchtungslicht mit parallelem Abbildungsstrahlengang durch das Mikroskop-Hauptobjektiv (101) zum Objektbereich (108) zu lenken
    **dadurch gekennzeichnet, dass**

    - eine Aufnahme (150) für einen zweiten Lichtleiter (151) eines OCT-Systems (152) vorgesehen ist, um einen OCT-Abtaststrahlengang (153) bereitzustellen; und
    - das Operationsmikroskop-Beleuchtungsmodul (120) ein zwischen der ersten Linsengruppe (125) und der zweiten Linsengruppe (126) angeordnetes Einkoppelelement (128) umfasst, das den OCT-Abtaststrahlengang (153) in den Beleuchtungsstrahlengang einkoppelt.

12. Operationsmikroskop-Beleuchtungsmodul nach Anspruch 11, **dadurch gekennzeichnet, dass** das Einkoppelelement (128) als Teilerspiegel, insbesondere als Planspiegel oder Teilerwürfel ausgebildet ist.

13. Operationsmikroskop-Beleuchtungsmodul nach Anspruch 11 oder Anspruch 12, **dadurch gekennzeichnet, dass** das Operationsmikroskop-Beleuchtungsmodul eine Einrichtung (156) zum Scannen des OCT-Abtaststrahlengangs umfasst.

14. Operationsmikroskop-Beleuchtungsmodul nach Anspruch 13, **dadurch gekennzeichnet, dass** die Einrichtung (156) zum Scannen des OCT-Abtaststrahlengangs einen ersten Scanspiegel (154) aufweist, der um eine erste Drehachse (303) bewegt werden kann.

15. Operationsmikroskop-Beleuchtungsmodul nach Anspruch 14, **dadurch gekennzeichnet, dass** ein zweiter Scanspiegel (155) vorgesehen ist, der um eine zweite Drehachse (304) bewegt werden kann, wobei die erste Drehachse (303) und die zweite Drehachse (304) seitlich versetzt in einem rechten Winkel zueinander stehen.

16. Operationsmikroskop-Beleuchtungsmodul nach einem der Ansprüche 11 bis 14, **dadurch gekennzeichnet, dass** das OCT-System einen Lichtleiter (151) umfasst, der einen Lichtaustrittsabschnitt für den OCT-Abtaststrahlengang aufweist, wobei Mittel zum Bewegen des Lichtaustrittsabschnitts (174) des Lichtleiters (151) vorgesehen sind.

17. Operationsmikroskop-Beleuchtungsmodul nach einem der Ansprüche 11 bis 16, **dadurch gekennzeichnet, dass** das OCT-System eine Kollimationsoptik (157) umfasst, welche den OCT-Abtaststrahlengang mit der zweiten Linsengruppe (126) der Beleuchtungsoptik in einen im wesentlichen parallelen Abtaststrahlengang überführt.

18. Operationsmikroskop-Beleuchtungsmodul nach Anspruch 17, **dadurch gekennzeichnet, dass** für das Einstellen der OCT-Abtastebene (170) des OCT-Systems (152) Mittel zum Bewegen (171) der Kollimationsoptik (157) vorgesehen sind.

## Claims

1. Operation microscope (100)

   - with an observation beam path (105) for examining an object region (108);
   - with an illumination device (120) comprising an illumination optical system, which images a field stop (124) illuminated by a light source as a parallel illumination beam path to infinity;
   - in which the illumination optical system has a first lens group (125) and a second lens group (126);
   - with an objective (101) arranged in the parallel illumination beam path in order to image the field stop (124) onto the object region (108);
   - with an OCT system (152) for examining the object region (108);
   - in which the OCT system (152) comprises an OCT scanning beam path (153), which is guided through the objective (101);
   **characterized in that**
   - a coupling-in element (128) is provided in the illumination beam path between the first lens group (125) and the second lens group (126), the element coupling the OCT scanning beam path into the illumination beam path.

2. Operation microscope according to Claim 1, **characterized in that** the coupling-in element (128) is designed as a splitter mirror, in particular as a plane mirror or a splitter cube.

3. Operation microscope according to Claim 1 or 2, **characterized in that** the objective arranged in the parallel observation beam path is designed as a main objective (101) of the microscope and penetrated by the observation beam path (105, 105a, 105b) of the operation microscope (100).

4. Operation microscope according to Claim 3, **characterized in that** a deflection device (129, 130) is provided on the side of the objective (101) facing away from the object, and it deflects the illumination light of the illumination device to the objective (101).

5. Operation microscope according to Claim 4, **characterized in that** the deflection device is designed as a beam splitter, which is penetrated by the observation beam path of the operation microscope.

6. Operation microscope according to one of Claims 1 to 5, **characterized in that** the OCT system for scanning the OCT scanning beam path comprises a first scanning mirror (154), which can be moved about a first rotational axis (303).

7. Operation microscope according to Claim 6, **characterized in that** provision is made for a second scanning mirror (155), which can be moved about a second rotational axis (304), with the first rotational axis (303) being laterally offset and at a right angle to the second rotational axis (304).

8. Operation microscope according to one of Claims 1 to 7, **characterized in that** the OCT system comprises an optical waveguide (151), which has a light-emission section for the OCT scanning beam path, in which provision is made for means for moving the light-emission section (174) of the optical waveguide (151).

9. Operation microscope according to one of Claims 1 to 8, **characterized in that** the OCT system comprises a collimation optical system (157), which, with the second lens group (126) of the illumination optical system, transfers the OCT scanning beam path into a substantially parallel scanning beam path.

10. Operation microscope according to Claim 9, **characterized in that** provision is made for means for moving (171) the collimation optical system (157) in order to set the OCT scanning plane (170) of the OCT system (152).

11. Operation-microscope illumination module (120) to be connected to an operation microscope (100)

    - with a receptacle (121) for a first optical waveguide (122) in order to provide illumination light (123);
    - with a field stop (124), which can be illuminated by light from the optical waveguide (122);
    - with an illumination optical system, which images the field stop (124) as a parallel imaging beam path to infinity; in which
    - the illumination optical system has a first lens group (125) and a second lens group (126); and
    - with an illumination mirror (129, 130), used to guide the illumination light with the parallel imaging beam path, emanating from the field stop (124), to the object region (108) through the main objective (101) of the microscope,
    **characterized in that**
    - provision is made for a receptacle (150) for a second optical waveguide (151) of an OCT system (152) in order to provide an OCT scanning beam path (153); and
    - the operation-microscope illumination module (120) comprises, arranged between the first lens group (125) and the second lens group (126), a coupling-in element (128), which couples the OCT scanning beam path (153) into the illumination beam path.

**12.** Operation-microscope illumination module according to Claim 11, **characterized in that** the coupling-in element (128) is designed as a splitter mirror, in particular as a plane mirror or a splitter cube.

**13.** Operation-microscope illumination module according to Claim 11 or Claim 12, **characterized in that** the operation-microscope illumination module comprises a device (156) for scanning the OCT scanning beam path.

**14.** Operation-microscope illumination module according to Claim 13, **characterized in that** the device (156) for scanning the OCT scanning beam path has a first scanning mirror (154), which can be moved about a first rotational axis (303).

**15.** Operation-microscope illumination module according to Claim 14, **characterized in that** provision is made for a second scanning mirror (155), which can be moved about a second rotational axis (304), with the first rotational axis (303) being laterally offset and at a right angle to the second rotational axis (304).

**16.** Operation-microscope illumination module according to one of Claims 11 to 14, **characterized in that** the OCT system comprises an optical waveguide (151), which has a light-emission section for the OCT scanning beam path, in which provision is made for means for moving the light-emission section (174) of the optical waveguide (151).

**17.** Operation-microscope illumination module according to one of Claims 11 to 16, **characterized in that** the OCT system comprises an collimation optical system (157), which, with the second lens group (126) of the illumination optical system, transfers the OCT scanning beam path into a substantially parallel scanning beam path.

**18.** Operation-microscope illumination module according to Claim 17, **characterized in that** provision is made for means for moving (171) the collimation optical system (157) in order to set the OCT scanning plane (170) of the OCT system (152).

**Revendications**

**1.** Microscope d'opération (100)

- avec un chemin optique d'observation (105) pour l'examen d'un espace objet (108) ;
- avec un dispositif d'éclairage (120) comportant une optique d'éclairage qui reproduit à l'infini un diaphragme du champ d'éclairage (124) illuminé par une source lumineuse, sous la forme d'un chemin optique d'éclairage parallèle ;
- dans lequel l'optique d'éclairage comporte un premier groupe de lentilles (125) et un deuxième groupe de lentilles (126) ;
- avec un objectif (101) disposé dans le chemin optique d'éclairage, pour reproduire le diaphragme du champ d'éclairage (124) dans l'espace objet (108) ;
- avec un système OCT (152) pour l'examen de l'espace objet (108) ;
- dans lequel le système OCT (152) comporte un chemin optique d'exploration OCT (153) qui est acheminé à travers l'objectif (101);
**caractérisé en ce que**
- dans le chemin optique d'éclairage entre le premier groupe de lentilles (125) et le deuxième groupe de lentilles (126), on prévoit un élément de couplage d'entrée (128) pour coupler le chemin optique d'exploration OCT à l'intérieur du chemin optique d'éclairage.

**2.** Microscope d'opération selon la revendication 1, **caractérisé en ce que** l'élément de couplage d'entrée (128) est réalisé sous la forme d'un miroir diviseur, notamment d'un miroir plan ou d'un cube diviseur.

**3.** Microscope d'opération selon la revendication 1 ou 2, **caractérisé en ce que** l'objectif disposé dans le chemin optique d'observation parallèle est réalisé sous la forme de l'objectif principal du microscope (101) et qu'il est traversé par le chemin optique d'observation (105, 105a, 105b) du microscope d'opération (100).

**4.** Microscope d'opération selon la revendication 3, **caractérisé en ce qu'**un dispositif de déviation (129, 130) est prévu sur la face de l'objectif (101) opposée à l'objet pour dévier la lumière d'éclairage du dispositif d'éclairage vers l'objectif (101).

**5.** Microscope d'opération selon la revendication 4, **caractérisé en ce que** le dispositif de déviation est réalisé sous la forme d'un diviseur optique traversé par le chemin optique d'observation du microscope d'opération.

**6.** Microscope d'opération selon une des revendications 1 à 5, **caractérisé en ce que** le système OCT pour le balayage du chemin optique d'exploration OCT comporte un premier miroir de balayage (154) mobile autour d'un premier axe de rotation (303).

**7.** Microscope d'opération selon la revendication 6, **caractérisé en ce qu'**un deuxième miroir de balayage (155) mobile autour d'un deuxième axe de rotation (304) est prévu, le premier axe de rotation (303) et le deuxième axe de rotation (304) étant mutuellement perpendiculaires décalés latéralement.

8. Microscope d'opération selon une des revendications 1 à 7, **caractérisé en ce que** le système OCT comporte un conduit de lumière (151) qui présente une section de sortie de lumière pour le chemin optique d'exploration OCT, des moyens étant prévus pour mouvoir la section de sortie de lumière (174) du conduit de lumière (151).

9. Microscope d'opération selon une des revendications 1 à 8, **caractérisé en ce que** le système OCT comporte une optique de collimation (157) qui transforme le chemin optique d'exploration OCT avec le deuxième groupe de lentilles (126) de l'optique d'éclairage en un chemin optique d'exploration essentiellement parallèle.

10. Microscope d'opération selon la revendication 9, **caractérisé en ce que** des moyens (171) pour mouvoir l'optique de collimation (157) sont prévus pour le réglage du plan de balayage OCT (170) du système OCT (152).

11. Module d'éclairage de microscope d'opération (120) pour le raccordement à un microscope d'opération (100)

    - avec un accueil (121) pour un premier conduit de lumière (122) pour recevoir la lumière d'éclairage (123);
    - avec un diaphragme du champ d'éclairage (124) qui peut être illuminé par de la lumière issue du conduit de lumière (122)
    - avec une optique d'éclairage qui reproduit à l'infini le diaphragme du champ d'éclairage (124) sous la forme d'un chemin optique d'éclairage parallèle ; dans lequel
    - l'optique d'éclairage comporte un premier groupe de lentilles (125) et un deuxième groupe de lentilles (126) ; et
    - avec un miroir d'éclairage (129, 130) servant à diriger la lumière d'éclairage sortant du diaphragme du champ d'éclairage (124) avec un chemin optique de reproduction parallèle, à travers l'objectif principal du microscope (101) vers l'espace objet (108) **caractérisé en ce que**
    - un accueil (150) est prévu pour un deuxième conduit de lumière (151) d'un système OCT (152) pour recevoir un chemin optique d'exploration OCT (153), et
    - que le module d'éclairage de microscope d'opération (120) comporte entre le premier groupe de lentilles (125) et le deuxième groupe de lentilles (126), un élément de couplage d'entrée (128) pour coupler le chemin optique d'exploration OCT (153) à l'intérieur du chemin optique d'éclairage.

12. Module d'éclairage de microscope d'opération selon la revendication 11, **caractérisé en ce que** l'élément de couplage d'entrée (128) est réalisé sous la forme d'un miroir diviseur, notamment d'un miroir plan ou d'un cube diviseur.

13. Module d'éclairage de microscope d'opération selon la revendication 11 ou la revendication 12, **caractérisé en ce que** le module d'éclairage de microscope d'opération comporte un dispositif (156) pour le balayage du chemin optique d'exploration OCT.

14. Module d'éclairage de microscope d'opération selon la revendication 13, **caractérisé en ce que** le dispositif (156) pour le balayage du chemin optique d'exploration OCT comporte un premier miroir de balayage (154) mobile autour d'un premier axe de rotation (303).

15. Module d'éclairage de microscope d'opération selon la revendication 14, **caractérisé en ce qu'**un deuxième miroir de balayage (155) mobile autour d'un deuxième axe de rotation (304) est prévu, le premier axe de rotation (303) et le deuxième axe de rotation (304) étant mutuellement perpendiculaires décalés latéralement.

16. Module d'éclairage de microscope d'opération selon une des revendications 11 à 14, **caractérisé en ce que** le système OCT comporte un conduit de lumière (151) qui présente une section de sortie de lumière pour le chemin optique d'exploration OCT, des moyens étant prévus pour mouvoir la section de sortie de lumière (174) du conduit de lumière (151).

17. Module d'éclairage de microscope d'opération selon une des revendications 11 à 16, **caractérisé en ce que** le système OCT comporte une optique de collimation (157) qui transforme le chemin optique d'exploration OCT avec le deuxième groupe de lentilles (126) de l'optique d'éclairage en un chemin optique d'exploration essentiellement parallèle.

18. Module d'éclairage de microscope d'opération selon la revendication 17, **caractérisé en ce que** des moyens (171) pour mouvoir l'optique de collimation (157) sont prévus pour le réglage du plan de balayage OCT (170) du système OCT (152).

# FIG.1

EP 1 918 756 B1

# FIG.2

# FIG.3

12

## FIG.4

## FIG.5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0815801 A
- US 5321501 A
- WO 2006100544 A1